# EUROPEAN PATENT SPECIFICATION

(11) **EP 3 636 211 B1**
(45) Date of publication and mention of the grant of the patent: **14.04.2021**
(21) Application number: 18200195.8
(22) Date of filing: 12.10.2018
(51) Int. Cl.: A61C 13/00

(54) **METHOD FOR PLANNING A RESTORATIVE DENTAL TREATMENT**
VERFAHREN ZUR PLANUNG EINER RESTAURATIVEN ZAHNBEHANDLUNG
PROCÉDÉ DE PLANIFICATION D'UN TRAITEMENT DENTAIRE DE RESTAURATION

(43) Date of publication of application: 15.04.2020
(73) Proprietor: Sirona Dental Systems GmbH, 64625 Bensheim (DE); DENTSPLY SIRONA Inc., York, 17401-2991 (US)
(72) Inventor: SCHNEIDER, Sascha, 64367 Mühltal (DE); DERZAPF, Evgenij, 64653 Lorsch (DE)
(74) Representative: Özer, Alpdeniz

(56) References cited:
- WO-A1-2013/164410
- US-A1- 2011 038 514
- US-B2- 7 403 830

## Description

### Technical Field

The present invention relates to a method for planning a restorative dental treatment for a patient as well as a computer program for implementing this method and a machine-readable data storage medium on which such a program is stored.

### State of the Art

A restorative dental treatment, wherein teeth of the patient are partially or completely replaced by dental replacement products, for example crowns, bridges, onlays, inlays or attachments (removable tooth replacement), is often necessary in dentistry. Digital processes are known for the design and the creation of such restorations, whereby newer digital methods work with computer-assisted design and, if applicable, computer-assisted production of the restorations (CAD/CAM technology) to provide optimum restorations for the patient, for example on the basis of tooth libraries or biogenerically calculated tooth models.

With a conventional, analog production of restorations, which use an arrangement of layers on a plaster model for example, if there are multiple restorations, i.e. multiple dental replacement products, the dental technician or the manufacturer of the restorations inevitably encounters the problem of how to insert the products into the dentition or the mouth of the patient in an optimum manner. Since the dental technician is directly confronted with this sometimes problematic step during the production of the restorations, he can avoid problems later during the insertion of the restorations on the patient by making the appropriate adjustments to the model ahead of time. Since this manual step in the production of the restorations is generally omitted in the age of digital tooth models, the problems may not occur until the dental replacement products are inserted, so that the treating dentist then spontaneously has to help himself by reworking and remaking, or by grinding down the restorations or, if necessary, even the existing teeth

Document US7403830 B2 discloses a computerized production of data records used in examination of artificial dentures, involves using computer to create data record representing artificial denture or part of artificial denture for fabrication of artificial denture, the method comprising transmitting the preparation instruction to at least one of a dentist and a dental technician.

The US patent application US 2016/0242880 A1 addresses the insertion of a number of artificial teeth or restorations. It describes a computer-implemented method for designing the restorations, wherein the shape of the adjacent tooth replacement is adjusted during modeling if another tooth is modified in the digital model.

The international patent application WO 2013/164410 A1 describes a digital method for designing a 3D model for a tooth replacement, wherein the insertion location in the patient's dentition is also represented in the 3D model. The insertion path for the tooth replacement is determined, and the 3D model of the tooth replacement is adjusted in such a way that undercuts with respect to the adjacent teeth, for example, are accounted for in the calculation of the shape of the restoration to be inserted.

In light of that, the object of the invention is to simplify and improve the insertion of dental restorations into the mouth of a patient, wherein problems during the insertion of multiple dental replacement products during the treatment are to be avoided.

### Presentation of the Invention

This task is achieved with a method for planning a restorative dental treatment, as is the subject matter of the main claim. Preferred embodiments of this method, as well as a computer program for carrying out the method and a machine-readable data storage medium result from the further claims.

In the method for planning a restorative dental treatment for a patient at least two dental restorations are digitally constructed and an optimum specification for the insertion of the restorations into the mouth of the patient is calculated, wherein the optimum specification for the insertion of the restorations comprises an optimum sequence of the restorations to be inserted, if applicable including optimum insertion paths for the restorations to be inserted. The method according to the invention, which can be carried out for example within the framework of a CAD/CAM program, in particular comprises the following steps:
a. calculation/construction of geometries for the restorations,
b. identification of potential conflicts for possible insertion sequences and, if applicable, insertion paths, and
c. output of an available optimum specification for the insertion of the restorations, wherein the optimum specification allows an insertion of the restorations without conflicts or, if applicable, a repetition of steps a. - c., if no optimum specification without conflicts is possible.

An optimum specification for the insertion of the restorations is to be understood as a specification with which, when accounted for by the practitioner, no problems or conflicts are to be expected for the insertion of the restorations into the patient's mouth. Interfering contacts of the restorations with one another and with the adjacent tooth situation are in particular avoided with an optimum specification. The determination of the optimum specification can furthermore account for the restorations being able to be inserted quickly and straightforwardly.

The advantage of this method is that the user no longer has to design and adjust the restorations manually, for example by looking at a scan or directly into the mouth of the patient or using an analog model, to make them fit. Instead, an optimal insertion of the restorations with respect to the sequence and, if applicable, also with respect to the insertion paths is planned prior to the insertion of the restorations and provided as a recommendation for the treating dentist, so that a problem-free and quick insertion of the tooth restorations into the dentition of the patient is possible, even for complicated geometries of the restorations, for example undercuts and the like. A trial and error approach to the insertion of the restorations and a subsequent adjustment, for example by grinding down the restorations themselves or even the existing teeth in the dentition of the patient, is no longer necessary, thus avoiding discomfort and disadvantages for the patient. This also facilitates the work of the treating dentist considerably, because, with the method according to the invention, he can be provided with an optimum specification for the insertion of the restorations in the form of a guide as the best possible recommendation, in which potential problems are accounted for in advance and avoided by appropriate adjustments of the insertion sequence and the insertion paths for the specific treatment.

Until now, the user has had to "practice" on a model and make himself a corresponding insertion protocol or use a manual trial and error approach for the insertion into the patient's mouth. Depending on the number of restorations, and with it the variations of the insertion sequence, this inevitably leads to complications; in particular if a cement or adhesive that hardens over time is used, and a decision potentially has to be made about which stump to coat with cement or adhesive next. Blocking out problematic clamping points with respect to the adjacent teeth in the contact region, for example during insertion with the hand-held instrument, was another variation for the conventional insertion of a plurality of dental replacement products. This variation inevitably resulted in suboptimal and unnatural tooth shapes in the contact region and could cause problems for the patient. The method according to the invention avoids all these disadvantages by calculating an optimum specification for the insertion of the restorations prior to the actual treatment of the patient and making it available to the user, i.e. the treating dentist, so that the insertion of the restorations can be carried out smoothly and without any problems.

The insertion paths, which can be accounted for during the insertion of the restorations along with the actual sequence, can be straight and/or curved. The insertion paths can furthermore also comprise rotations. A rotation of the restoration during insertion can be very helpful, for example in order to avoid problems in the event of undercuts. The rotations are therefore preferably accounted for in the determination of the insertion sequence according to the method according to the invention. Different possible rotations can be automatically checked in the course of the method, to test whether they are helpful for the insertion. The possibly necessary rotations during the insertion of the restorations can thus be included in the specification or in a guide or description for the treating dentist. This can also be interesting and helpful for inlays or other dental restorations.

The insertion paths further preferably also comprise a definition of the insertion directions. The specification for the insertion of the restorations can therefore also comprise an automatic attachment direction determination based on the insertion direction of the affected restorations.

The calculation of the optimum specification for the insertion of the restorations preferably accounts for the restorations to be inserted not being deformed, in particular not deformed counter to their optimal shape. One particular advantage of the method according to the invention is therefore that the specification for the insertion of the restorations takes place under the premise that optimally shaped restorations are inserted. The optimum tooth shape can consequently be made possible by the restorations, even in the event of complicated configurations in the contact region. If a specification for the insertion of the restorations is not possible for the optimum restoration shape, however, it is also possible to perform adjustments with feedback from the design of the geometries of the restorations. This means that, if the geometries of the tooth replacement originally determined to be optimum cannot practically be inserted into the mouth, adjustments can be made to the geometries. The optimum specification for the insertion is subsequently determined under these new conditions, so that the tooth replacement can be inserted in a specific sequence without interfering contacts. The insertion sequence and, if applicable, the optimum insertion paths needed to achieve this can be calculated automatically in an iterative process.

Other factors can also play a role in the adjustments during construction, and in general in the construction of the restorations. In the case of abutments, for example, consideration can be given to the fact that, as a rule, one-piece abutment solutions are advantageous compared to multipart abutments and should therefore be preferred.

Furthermore, in the method according to the invention, external constraints for the insertion directions for the restorations to be inserted can be accounted for in the calculation of the optimum specification for the insertion of the restorations, wherein the insertion directions are to be understood as a part of the insertion paths. Fixedly predefined implant positions or attachment parts, for example, which affect or specify the possible insertion directions of the restorations, are accounted for in the calculation of the optimum specification.

Technical limitations can also be accounted for in the construction of the restorations, for example an automatic consideration of machine limits, for example of a four-axle milling/grinding machine, wherein the number of axes of a milling/grinding machine may limit the possible shapes of the restoration bodies that can be produced, e. g. the possibility of undercuts.

It is further possible for the optimum specification for the insertion of the restorations derived according to the method to be modified manually and a new optimum specification to subsequently created taking into account the modifications. The treating dentist can thus enter practical limitations that arise during the treatment into the system, for example, so that an appropriately adjusted specification for the insertion of the restorations can be calculated and issued. For example, the dentist could identify the first tooth to be inserted, and the system, i.e. the method according to the invention, would adjust the planning for the insertion of the restorations according to that specification.

The method according to the invention can in principle be used for all types of restorations and attachments, for example for onlays with contact in the interproximal region, for crowns, for abutments or for bridges with adjacent crowns. The method according to the invention can also be used advantageously for veneers with interproximal contact.

Overall, the method offers the advantage for the user, i.e. in particular the treating dentist, and therefore obviously also the patient, that problems during the insertion of the tooth replacement or of the restorations are avoided. Insertion conflicts are recognized in advance and automatically accounted for by the method, so that the safety of the user and the patient is increased and discomfort and problems are avoided.

The method according to the invention presupposes that the specification for the insertion of the restorations concerns at least two restorations. In this configuration of the method, if an optimum specification for the insertion of the restorations cannot be calculated, the geometries for the restorations are accounted for in a manner involving feedback, perhaps by adjusting the geometries of the restorations. This means that, in this case, an insertion of the restorations into the dentition of the patient without a deformation of the restorations counter to their optimum shape, or possibly the surrounding teeth, would not be possible. The geometries then have to be adjusted to avoid later problems during the insertion of the restorations.

The described method is preferably carried out within the framework of a computer-assisted design program and/or production program for the planning and/or production of dental restorations (CAD/CAM systems). The method can, in a manner of speaking, automatically generate an optimum specification for the insertion of the restorations, wherein this specification can be provided as a guide or as a detailed description of the insertion of the restorations to the practitioner. This can be done in printable form, for example, or as a display on the screen of a computer or, for example, a mobile device.

The invention further comprises a computer program that is configured for implementing the described method. The invention further comprises a machine-readable data storage medium, on which such a computer program is stored.

Additional features and advantages of the invention emerge from the following description of design examples in conjunction with the drawings.

### Brief Description of the Drawings

The drawings show:
- Figure 1: a schematic flow chart of the method according to the invention in a preferred configuration;
- Figure 2: an illustration of adjustments in the construction of the restorations in problematic insertion situations and
- Figure 3: an illustration of a modification of the insertion paths in problematic insertion situations.

### Design Examples

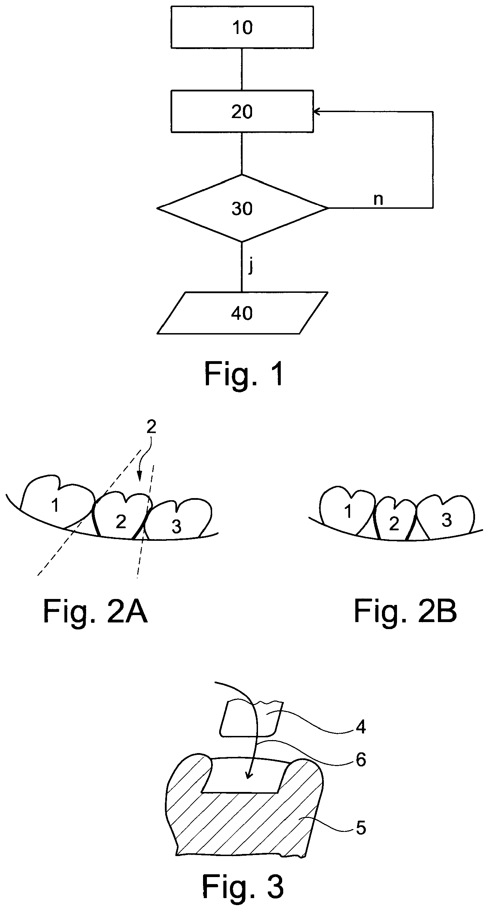

**Fig. 1** schematically illustrates the sequence of an embodiment of the method for planning a restorative dental treatment, wherein an optimum specification for the insertion of the restorations is determined. After the start 10 of the method, which is in particular integrated into a CAD/CAM system, geometries for the dental restorations are calculated and constructed in a per se known manner in Step 20. The subsequent query 30 determines whether potential conflicts are to be expected for possible insertion sequences and, if applicable, for possible insertion paths. The calculation of an optimum specification for the insertion sequence in particular accounts for external constraints, for example, implant insertion directions or already existing attachments, and accounts for adjacent tooth contacts. When identifying potential conflicts, for example, all possible sequences for the insertion of the restorations can be determined first and the insertion thereof can be simulated, for example, on a stump situation. An examination of the tangent direction determined by the interproximal contact situation, for example, can be conducted as well. A workable insertion sequence can thus be identified; if necessary taking into account specific insertion paths (rotations, insertion directions, etc.). If different paths are possible, one particularly suitable and advantageous specification, which can be particularly simply and quickly realized by the user, for example, can be selected. The optimum specification will be output by the system as a guide for the user, for example using sketches and/or in written form, in Step 40.

If a number of workable variants are obtained for the specification for inserting the restorations, a number of guides can be output that can be used alternatively. If the query in Step 30 shows that there is no optimum specification or that the insertion of the restorations under the given conditions is not possible without further adjustments, it is possible to return to Step 20 and carry out an adjustment of the geometries. The algorithm then starts over from the beginning, wherein one or more of the restorations can be adjusted accordingly to make an unproblematic insertion of the restorations into the mouth of the patient under new conditions possible.

This proposal calculation for the insertion of the restorations that accounts for the different options for the insertion sequence, and, if applicable, the insertion paths or insertion directions, ensures that the dentist can use the best possible tooth shapes, even if the contact situation is unfavorable. According to the invention, a particular insertion sequence is calculated and specified to avoid the need to compromise with respect to the tooth shape and the contact regions. Therefore, the optimum restoration shape does not have to be compromised for reasons of insertability, because an indirect insertion sequence, which is sometimes required as a result of the tooth shape, can be calculated and implemented in practice. The method allows the automatic generation of a guide for the treating dentist with the insertion sequence and, if applicable, the specific insertion paths automatically derived by the system, which the treating dentist can use as basis for the treatment, for example on a screen or as a printout.

The method can also be used, for example for the calculation of abutments (implant structures), to decide whether a one-piece abutment solution or a two-part abutment solution is most suitable for the case in question. Because of the additional cement or adhesive gaps that can thereby be avoided, one-piece abutment solutions are generally preferred. Problems can arise during the insertion of one-piece abutment solutions, however, which can be modeled and recognized in advance using the method according to the invention. To solve the problem, a possibly indirect insertion sequence for the restorations including the abutment can be calculated using the method according to the invention. If no suitable insertion sequence or suitable specification for the insertion of the restorations including the one-piece abutment is possible, a two-part abutment solution can be used, if appropriate.

**Fig. 2A****, B** schematically illustrates a difficult insertion situation with a strongly inclined curve of Spee, wherein the curve of Spee describes an arc which connects the cutting edges and cusps in the dentition (sagittal compensation curve). The curve of Spee, which must be taken into account in restorations for functional reasons, can lead to problematic insertion situations (Sub-figure A). These problems (undercuts) are identified in the simulation of the insertion of the restorations (order of the teeth 1, 2, 3) during the simulation within the scope of the method according to the invention and, in this case, cannot even be corrected by varying the insertion sequence. In this case, the conflicts are accounted for in advance within the scope of the method according to the invention, with adjustments to the geometry of the restorations (Tooth 2) (Sub-figure B).

**Fig. 3** illustrates the case in which problematic situations can be solved by varying the insertion paths. Due to the undercut situation of the inlay 4 to be inserted into the natural tooth 5, direct insertion in a straight line is not possible. The insertion is only possible with a rotation of the inlay 4 to be inserted. This situation can, in a similar manner, be applied to situations with a plurality of restorations.

## Claims

1. A computer-implemented method for planning a restorative dental treatment for a patient, wherein at least two dental restorations are digitally constructed (20) and a specification (40) for the insertion of the restorations into the mouth of the patient is calculated, wherein the specification for the insertion of the restorations comprises an order of the restorations to be inserted, if applicable including insertion paths for the restorations to be inserted, **characterized in that** the method comprises the following steps:
a. calculation of geometries for the restorations (20),
b. identification of potential conflicts for possible insertion sequences and, if applicable, insertion paths (30), and
c. output of an available optimum specification (40) for the insertion of the restorations, wherein the optimum specification allows an insertion of the restorations without conflicts, or repetition of steps a. - c., if no optimum specification is possible.

2. The computer-implemented method according to Claim 1, **characterized in that** the insertion paths are straight and/or curved.

3. The computer-implemented method according to Claim 1 or Claim 2, **characterized in that** the insertion paths comprise rotations.

4. The computer-implemented method according to any one of the preceding Claims, **characterized in that** the insertion paths comprise insertion directions.

5. The computer-implemented method according to any one of the preceding Claims, **characterized in that** the calculation of the optimum specification (40) for the insertion of the restorations accounts for the restorations to be inserted not being deformed.

6. The computer-implemented method according to any one of the preceding Claims, **characterized in that** the calculation of the optimum specification (40) for the insertion of the restorations accounts for a possible adjustment in the construction of the geometries of the restorations.

7. The computer-implemented method according to any one of the preceding Claims, **characterized in that**, in the construction of the restorations with abutments, one-piece abutments are preferred to multipart abutments.

8. The computer-implemented method according to any one of the preceding Claims, **characterized in that** the method is carried out within the framework of a computer-assisted design program and/or production program for the planning and/or production of dental restorations.

9. Computer program comprising instructions which, when the program is executed by a computer, cause the computer to carry out the method according to any one of Claims 1 to 8.

10. Machine-readable data storage medium, on which a computer program according to Claim 9 is stored.

## Patentansprüche

1. Computerimplementiertes Verfahren zur Planung einer restaurativen Zahnbehandlung für einen Patienten, wobei mindestens zwei Zahnrestaurationen digital aufgebaut sind (20), und eine Spezifikation (40) für das Einsetzen der Restaurationen in den Mund des Patienten berechnet wird, wobei die Spezifikation für das Einsetzen der Restaurationen eine Reihenfolge der Restaurationen, die einzusetzen sind, umfasst, die falls zutreffend, Einsetzwege für die Restaurationen, die einzusetzen sind, beinhaltet, **dadurch gekennzeichnet, dass** das Verfahren die folgenden Schritte umfasst:
a. Berechnung von Geometrien für die Restaurationen (20),
b. Identifizierung potenzieller Konflikte für mögliche Einsetzsequenzen und, falls zutreffend, Einsetzwege (30), und
c. Ausgabe einer verfügbaren optimalen Spezifikation (40) für das Einsetzen der Restaurationen, wobei die optimale Spezifikation ein Einsetzen der Restaurationen ohne Konflikte oder Wiederholung von Schritten a. bis c. erlaubt, falls keine optimale Spezifikation möglich ist.

2. Computerimplementiertes Verfahren nach Anspruch 1, **dadurch gekennzeichnet, dass** die Einsetzwege gerade und/oder gekrümmt sind.

3. Computerimplementiertes Verfahren nach Anspruch 1 oder Anspruch 2, **dadurch gekennzeichnet, dass** die Einsetzwege Rotationen umfassen.

4. Computerimplementiertes Verfahren nach einem der vorstehenden Ansprüche, **dadurch gekennzeichnet, dass** die Einsetzwege Einsetzrichtungen umfassen.

5. Computerimplementiertes Verfahren nach einem der vorstehenden Ansprüche, **dadurch gekennzeichnet, dass** die Berechnung der optimalen Spezifikation (40) für das Einsetzen der Restaurationen berücksichtigt, dass die Restaurationen, die einzusetzen sind, nicht verformt werden.

6. Computerimplementiertes Verfahren nach einem der vorstehenden Ansprüche, **dadurch gekennzeichnet, dass** die Berechnung der optimalen Spezifikation (40) für das Einsetzen der Restaurationen eine mögliche Anpassung der Konstruktion der Geometrien der Restaurationen berücksichtigt.

7. Computerimplementiertes Verfahren nach einem der vorstehenden Ansprüche, **dadurch gekennzeichnet, dass** bei der Konstruktion der Restaurationen mit Abutments einteilige Abutments gegenüber mehrteiligen Abutments bevorzugt werden.

8. Computerimplementiertes Verfahren nach einem der vorstehenden Ansprüche, **dadurch gekennzeichnet, dass** das Verfahren innerhalb des Rahmens eines computergestützten Konzeptionsprogramms und/oder Herstellungsprogramms zur Planung und/oder Herstellung von Zahnrestaurationen ausgeführt wird.

9. Computerprogramm, das Anweisungen umfasst, die, wenn das Programm von einem Computer ausgeführt wird, den Computer veranlassen, das Verfahren nach einem der Ansprüche 1 bis 8 auszuführen.

10. Maschinenlesbares Datenspeichermedium, auf dem ein Computerprogramm nach Anspruch 9 gespeichert ist.

## Revendications

1. Procédé mis en œuvre par ordinateur de planification d'un traitement dentaire de restauration pour un patient, dans lequel au moins deux restaurations dentaires sont construites numériquement (20) et une spécification (40) pour l'insertion des restaurations dans la bouche du patient est calculée, dans lequel la spécification pour l'insertion des restaurations comprend un ordre des restaurations à insérer, le cas échéant incluant des chemins d'insertion pour les restaurations à insérer, **caractérisé en ce que** le procédé comprend les étapes suivantes :
a. calcul des géométries des restaurations (20),
b. identification des conflits potentiels pour d'éventuelles séquences d'insertion et, le cas échéant, des chemins d'insertion (30), et
c. sortie d'une spécification (40) optimale disponible pour l'insertion des restaurations, la spécification optimale permettant une insertion des restaurations sans conflits, ou répétition des étapes a. à c. si aucune spécification optimale n'est possible.

2. Procédé mis en œuvre par ordinateur selon la revendication 1, **caractérisé en ce que** les chemins d'insertion sont droits et/ou courbes.

3. Procédé mis en œuvre par ordinateur selon la revendication 1 ou la revendication 2, **caractérisé en ce que** les chemins d'insertion comprennent des rotations.

4. Procédé mis en œuvre par ordinateur selon l'une quelconque des revendications précédentes, **caractérisé en ce que** les chemins d'insertion comprennent des directions d'insertion.

5. Procédé mis en œuvre par ordinateur selon l'une quelconque des revendications précédentes, **caractérisé en ce que** le calcul de la spécification (40) optimale pour l'insertion des restaurations tient compte du fait que les restaurations à insérer ne sont pas déformées.

6. Procédé mis en œuvre par ordinateur selon l'une quelconque des revendications précédentes, **caractérisé en ce que** le calcul de la spécification (40) optimale pour l'insertion des restaurations tient compte d'un ajustement possible dans la construction des géométries des restaurations.

7. Procédé mis en œuvre par ordinateur selon l'une quelconque des revendications précédentes, **caractérisé en ce que**, dans la construction des restaurations avec piliers, les piliers en une partie sont préférés aux piliers en plusieurs parties.

8. Procédé mis en œuvre par ordinateur selon l'une quelconque des revendications précédentes, **caractérisé en ce que** le procédé est réalisé dans le cadre d'un programme de conception assistée par ordinateur et/ou d'un programme de production pour la planification et/ou la production de restaurations dentaires.

9. Programme informatique comprenant des instructions qui, lorsque le programme est réalisé par un ordinateur, amènent l'ordinateur à effectuer le procédé selon l'une quelconque des revendications 1 à 8.

10. Support de stockage de données lisible par machine, sur lequel est stocké un programme informatique selon la revendication 9.
